# EUROPEAN PATENT APPLICATION

(11) **EP 3 653 245 A1**
(43) Date of publication of application: **20.05.2020**
(21) Application number: 17917529.4
(22) Date of filing: 14.07.2017
(51) Int. Cl.: A61M 5/50

(54) **SAFETY SYRINGE**

(71) Applicant: Hung, Chih-Hua, Hsinchu City 300 (TW)
(72) Inventor: Hung, Chih-Hua, Hsinchu City 300 (TW)
(74) Representative: Straus, Alexander
(86) International application number: PCT/CN2017/092891
(87) International publication number: WO 2019/010689

(57) **Abstract**

A safety syringe includes an outer barrel having a receiving groove located on an inner surface of a rear end thereof between a first annular flange and a second annular flange, an inner barrel having a needle mount located on a front end thereof and a positioning portion adjacent to the needle mount, and a plunger axially movably inserted into the inside of the inner barrel.

## Description

### BACKGROUND OF THE INVENTION

### 1. Field of the Invention

The present invention relates to safety syringes and more particularly, to such a safety syringe that has a simple structure and is easy to assemble.

### 2. Description of the Related Art

A syringe is a device to be used with a needle for injecting liquids such as medical fluid, blood or nutritional fluid into the human body. After the injection is completed, the needle is already contaminated with human blood, so the used needle must be safely processed to avoid medical staff or other people being injured by the needle. The early safe handling method was to insert the used needle into a needle cover for storage, but during the insertion process, the hands of the medical personnel may be accidentally pierced by the needle due to their carelessness or being pushed by other external forces, which in turn increases the risk of accidental infections for the medical personnel. Such problems also lead to so-called safety syringes to reduce the chance of medical personnel directly touching the needle with their hands.

As for the traditional safety syringe, after the medical personnel inserts the plunger into the outer barrel to complete the injection, the plunger will be locked with the needle holder through a special structural design, so that the needle holder with the needle can be pulled back into the outer barrel by the plunger. However, in the aforementioned prior art, the medical personnel needs to apply a large thrust to the plunger in order to form a snap connection between the plunger and the needle holder. This will not only cause medical personnel's assembly trouble, but if the force is too large, it may cause structural damage. Therefore, it is necessary to improve the structure of traditional safety syringes.

### SUMMARY OF THE INVENTION

The present invention has been accomplished under the circumstances in view. It is the main object of the present invention to provide a safety syringe, which has the characteristics of simple structure and easy assembly to increase convenience in use.

To achieve this and other objects of the present invention, a safety syringe comprises an outer barrel, an inner barrel, and a plunger. The outer barrel comprises a front end opening located in a front end thereof, a first rear end opening located in an opposing rear end thereof, a first annular flange and a second annular flange located on an inner surface of the rear end of the outer barrel, and a receiving groove located on the inner surface of the rear end of the outer barrel between the first annular flange and the second annular flange. The inner barrel comprises a needle mount located on a front end thereof, a positioning portion adjacent to the needle mount, a second rear end opening located in an opposing rear end thereof. The plunger is inserted through the second rear end opening of the inner barrel into the inside of the inner barrel and axially movable relative to the inner barrel. The inner barrel is inserted through the first rear end opening of the outer barrel into the inside of the outer barrel and axially movable relative to the outer barrel between a first position and a second position. When the inner barrel is located at the first position, the positioning portion of the inner barrel is located between the front end opening of the outer barrel and the second annular flange of the outer barrel so that the needle mount of the inner barrel is partially exposed to the outside of the outer barrel through the front end opening. When the inner barrel is located at the second position, the positioning portion of the inner barrel is located in the receiving groove of the outer barrel and blocked by the first annular flange and second annular flange of the outer barrel so that the needle mount of the inner barrel is completely inside the outer barrel.

It can be known from the above, when the inner barrel is located at the first position, the positioning portion of the inner barrel is located between the front end opening and second annular flange of the outer barrel, so that the inner barrel can be forced by an external force to move the needle mount partially out of the outer barrel through the front end opening, letting a needle in the needle mount of the inner barrel make an injection.

When the inner barrel is moved from the first position to the second position, the positioning portion of the inner barrel is located within the receiving groove of the outer barrel, and, the positioning portion of the inner barrel is blocked by the first annular flange and second annular flange of the outer barrel, so that the range of movement of the inner barrel is limited between the first annular flange and second annular flange of the outer barrel. At this time, the needle mount of the inner barrel and the needle are kept inside the outer barrel to increase safety after injection.

Preferably, the inner diameter of the second annular flange of the outer barrel gradually increases in direction away from the receiving groove of the outer barrel, so that when the inner barrel is being moved from the first position to the second position, the positioning portion can be moved over the second annular flange of the outer barrel with less effort.

Preferably, the first rear end opening of the outer barrel is adjacent to the first annular flange of the outer barrel, and the inner diameter of the first rear end opening of the outer barrel gradually increases in direction away from the first annular flange of the outer barrel, so that when the inner barrel is assembled in the outer barrel, the positioning portion can be moved over the first annular flange of the outer barrel with less effort.

Preferably, the axial length of the receiving groove of the outer barrel is larger than the axial length of the positioning portion of the inner barrel. When the inner barrel is located at the second position, a force can be applied to make the outer barrel skew so that the front end opening of the outer barrel and the needle mount of the inner barrel are staggered from each other and not on the same line. In this case, even the inner barrel or the plunger accidental collision, the needle will not extend through the front end opening of the outer barrel to the outside of the outer barrel, to further ensure that the needle has sufficient safety after injection.

Preferably, the positioning portion of the inner barrel is composed of four bumps equiangularly spaced around the central axis of the inner barrel. Alternatively, the positioning portion of the inner barrel can be an annular bump.

Other advantages and features of the present invention will be fully understood by reference to the following specification in conjunction with the accompanying drawings, in which like reference signs denote like components of structure.

### BRIEF DESCRIPTION OF THE DRAWINGS

FIG. 1 is a sectional exploded plain view of a safety syringe in accordance with a first embodiment of the present invention.
FIG. 2 is a sectional plain assembly view of the inner and outer barrels of the safety syringe in accordance with the first embodiment of the present invention, mainly illustrating the inner barrel in the second position.
FIG. 3 is similar to FIG. 2, mainly illustrating the inner barrel in the first position.
FIG. 4 is a top view of the inner barrel of the safety syringe in accordance with the first embodiment of the present invention.
FIG. 5 is a sectional assembly plain view of the safety syringe in accordance with the first embodiment of the present invention, mainly illustrating the needle protruded over the outer barrel.
FIG. 6 is similar to FIG. 5, illustrating the needle received inside the outer barrel.
FIG. 7 is similar to FIG. 6, illustrating the outer barrel skewed relative to the inner barrel.
FIG. 8 is a schematic sectional exploded view of a part of a safety syringe in accordance with a second embodiment of the present invention, illustrating an alternate form of the needle mount.
FIG. 9 is a schematic sectional exploded view of a part of a safety syringe in accordance with a third embodiment of the present invention, illustrating an alternate form of the positioning portion.
FIG. 10 is a top view of FIG. 9.

### DETAILED DESCRIPTION OF THE INVENTION

The following describes the technical content and features of the present invention in detail with the enumerated several preferred embodiments and the drawings. The directional adjectives of "front", "rear", "inside", "outside" and the like mentioned in the description of this specification are merely examples of descriptions based on the direction of normal use and are not intended to limit the scope of claims.

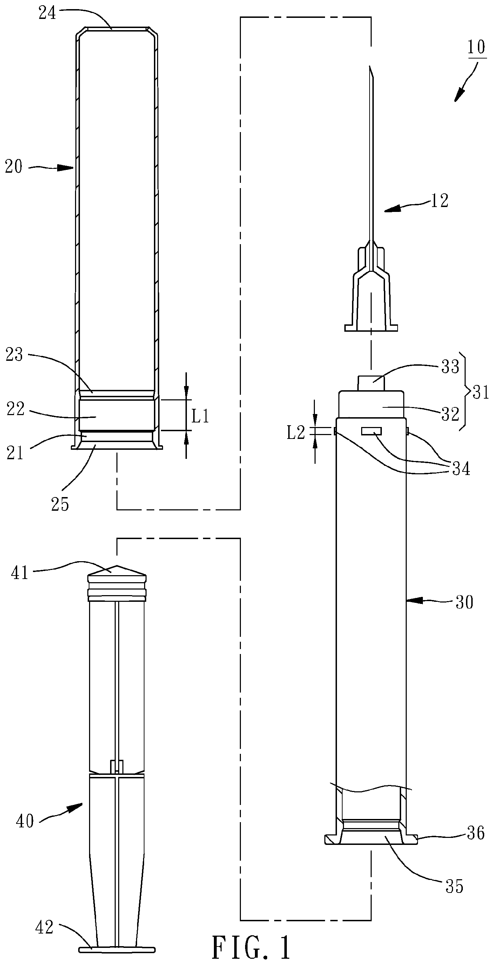

Referring to FIG. 1, a safety syringe **10** in accordance with a first embodiment of the present invention comprises an outer barrel **20,** an inner barrel **30,** and a plunger **40.**

The outer barrel **20** is made of plastic. In order from back to front, the inner wall surface of the rear end of the outer barrel **20** has a first annular flange **21,** a receiving groove **22** adjacent to the first annular flange **21,** and a second annular flange **23** adjacent to the receiving groove **22.** The second annular flange **23** gradually increases in the inner diameter in direction away from the receiving groove **22.** In addition, the outer barrel **20** has a front end opening **24** and a first rear end opening **25.** The first rear end opening **25** is adjacent to the first annular flange **21,** and the first rear end opening **25** gradually increases in the inner diameter in direction away from the first annular flange **21.**

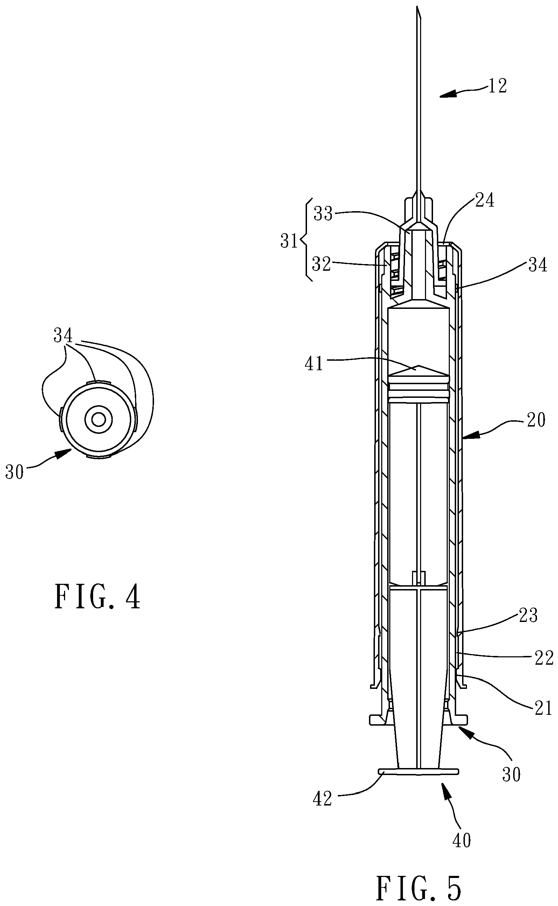

The inner barrel **30** is made of plastic. The front end of the inner barrel **30** has a needle mount **31** and a positioning portion **34** adjacent to the needle mount **31.** The needle mount **31** in this embodiment is composed of a screwing base **32** and an adapter shaft **33** located on the screwing base **32.** Therefore, when used with the needle **12,** the needle **12** is screwed to the screwing base **32** of the needle mount **31** of the inner barrel **30** and simultaneously attached onto the adapter shaft **33** of the needle mount **31** of the inner barrel **30.** As shown in FIG. 4, the positioning portion **34** in this embodiment is composed of four bumps that are equiangularly spaced around the central axis of the inner barrel **30.** The axial length **L2** of the positioning portion **34** is shorter than the axial length **L1** of the receiving groove **22** of the outer barrel **20.** In addition, the rear end of the inner barrel **30** has a second rear end opening **35** and a finger flange **36** outwardly extended from the periphery of the second rear end opening **35.**

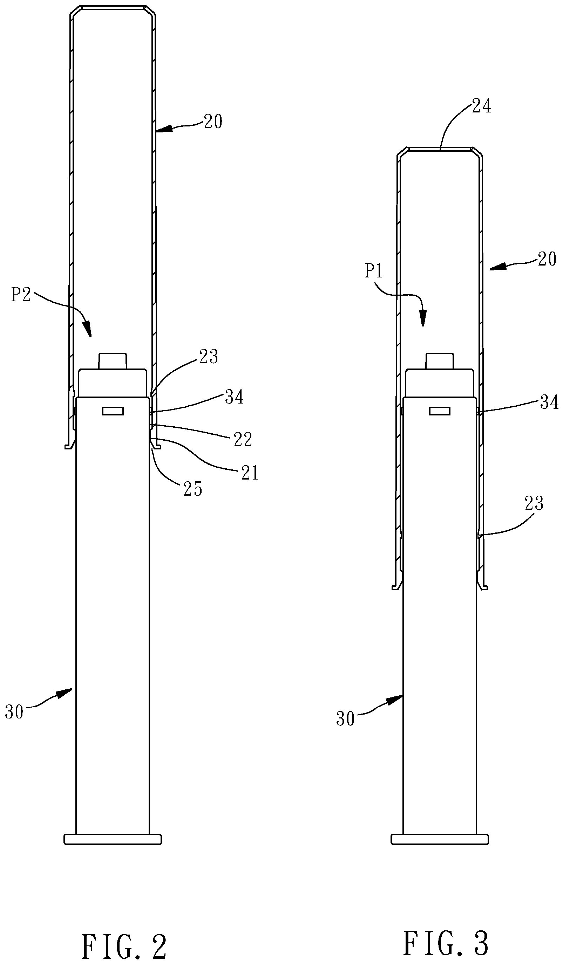

When the inner barrel **30** is assembled in the outer barrel **20,** the inner diameter of the first rear end opening **25** gradually increases in direction away from the first annular flange **21,** so the inner barrel **30** can easily be inserted through the first rear end opening **25** of the outer barrel **20** into the inside of the outer barrel **20.** During the process of inserting the inner barrel **30** into the outer barrel **20,** the positioning portion **34** of the inner barrel **30** will be squeezed to deform by the first annular flange **21** of the outer barrel **20.** Because the protruding height of the positioning portion **34** of the inner barrel **30** is limited, the positioning portion **34** of the inner barrel **30** can be moved over the first annular flange **21** of the outer barrel **20** into the receiving groove **22** of the outer barrel **20** without applying a large thrust to the inner barrel **30.** The inner barrel **30** at this time is located at a second position **P2** (see FIG. 2). When the inner barrel **30** is located at the second position **P2** as shown in FIG. 2, the positioning portion **34** of the inner barrel **30** is located within the receiving groove **22** of the outer barrel **20,** and the positioning portion **34** of the inner barrel **30** is blocked by the first annular flange **21** and second annular flange **23** of the outer barrel **20,** so that the movement range of the inner barrel **30** at this time is limited between the first annular flange **21** and second annular flange **23** of the outer barrel **20.** Then, continue to apply thrust to the inner barrel **30** to let the positioning portion **34** of the inner barrel **30** pass the second annular flange **23** of the outer barrel **20** to complete the assembly of the two. At this time, the inner barrel **30** is located at a first position **P1** (see FIG. 3). When the inner barrel **30** is located at the first position **P1** as shown in FIG. 3, the positioning portion **34** of the inner barrel **30** is located between the front end opening **24** of the outer barrel **20** and the second annular flange **23** of the outer barrel **20,** so that the inner barrel **30** can make a relatively large range of movement relative to the outer barrel **20.**

The plunger **40** has a push top **41** at a front end thereof and a thumb rest **42** at an opposing rear end thereof. The plunger **40** is inserted through the second rear end opening **35** of the inner barrel **30** into the inside of the inner barrel **30,** so that the push top **41** of the plunger **40** is located in the inner barrel **30.** At this time, the thumb rest **42** of the plunger **40** is located outside the inner barrel **30** for a medical staff to press.

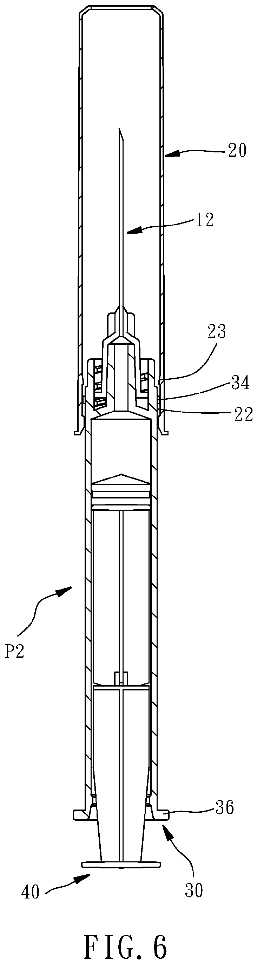

It can be known from the above that the inner barrel **30** is first maintained at the first position **P1** and then pushed forward, so that the screwing base **32** of the needle mount **31** of the inner barrel **30** abuts against the periphery of the front end opening **24** of the outer barrel **20,** as shown in FIG. 5. At this time, the adapter shaft **33** of the needle mount **31** of the inner barrel **30** will be partially exposed to the outside of the outer barrel **20** through the front end opening **24** of the outer barrel **20,** so that the needle **12** is exposed outside the outer barrel **20.** Then press the thumb rest **42** of the plunger **40,** so that the push top **41** of the plunger **40** is forced to push the medicinal solution out of the inner barrel **30** through the needle **12** for injection. After the injection is completed, pull the finger flange **36** of the inner barrel **30** to make the inner barrel **30,** along with the needle **12** and the plunger **40** move backward toward the second position **P2.** Since the inner diameter of the second annular flange **23** of the outer barrel **20** gradually increases in direction away from the receiving groove **22** of the outer barrel **20** and the protruding height of the positioning portion **34** of the inner barrel **30** is limited, it is not necessary to apply a large amount of pull force to the inner barrel **30** to move the positioning portion **34** of the inner barrel **30** over the second annular flange **23** of the outer barrel **20** to the receiving groove **22** of the outer barrel **20,** enabling the inner barrel **30** to be located at the second position **P2,** as shown in FIG. 6. At this time, the needle **12** is kept inside the outer barrel **20** to increase safety after the injection is completed.

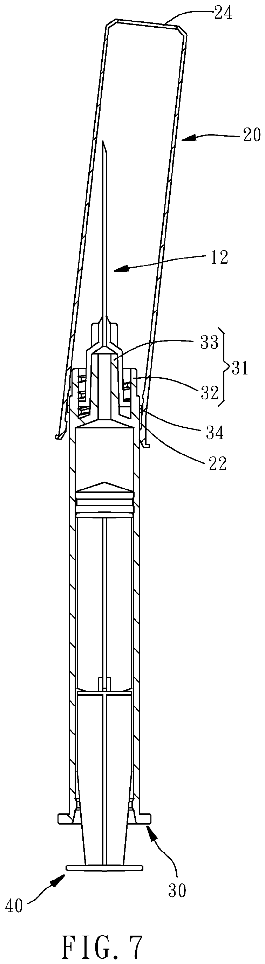

On the other hand, when the inner barrel **3**0 is located at the second position **P2** as shown in FIG. 6, based on the material characteristics of the outer barrel **20,** plus the relationship that the receiving groove **22** of the outer barrel **20** is larger in axial length than the positioning portion **34** of the inner barrel **30,** at this time, the outer barrel **20** can be further bent to skew relative to the inner barrel **30,** as shown in FIG. 7.

The main thing is to make the outer barrel **20** skew so that the front end opening **24** of the outer barrel **20** and the needle mount **31** of the inner barrel **30** are staggered from each other and not on the same line. In this case, even the inner barrel **30** or the plunger **40** accidental collision, the needle **12** will not extend through the front end opening **24** of the outer barrel **20** to the outside of the outer barrel **20,** to further ensure that the needle **12** has sufficient safety after injection.

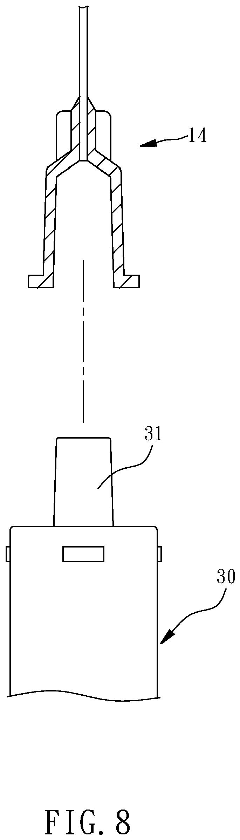

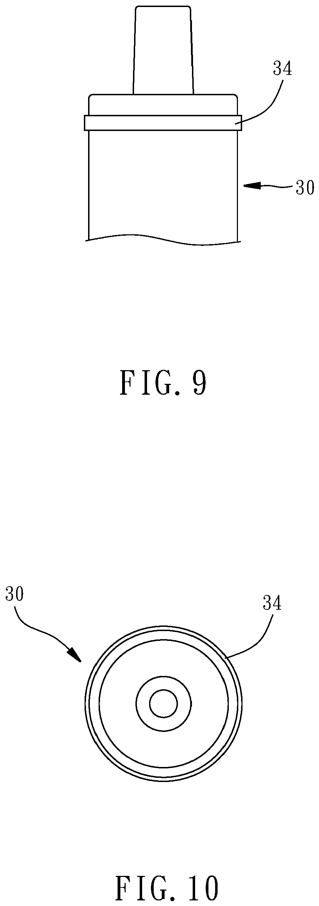

It should be added here that in order to cooperate with different forms of the needle **14,** the needle mount **31** of the inner barrel **30** can have different structural changes. As shown in FIG. 8, in the second embodiment of the present invention, the needle mount **31** is a single adapter shaft, and the needle **14** is assembled by directly attaching onto the single adapter shaft. In addition, as shown in FIGS. 9 and 10, the positioning portion **34** of the inner barrel **30** is not necessarily formed by four bumps. In the third embodiment of the present invention, the positioning portion **34** of the inner barrel **30** is a single annular bump, so that is can also achieve a snap-in effect with the first annular flange **21** and second annular flange **23** of the outer barrel **20.**

In conclusion, the safety syringe **10** of the present invention uses the cooperation between the positioning portion **34** of the inner barrel **30** and the receiving groove **22** of the outer barrel **20,** plus the position-limiting effect provided by the first annular flange **21** and second annular flange **23** of the outer barrel **20** to effectively simplify the structure compared to the prior art, and the overall assembly of the safety syringe **10** can be completed without much effort, thereby achieving the object of the present invention.

Of course, the present invention may have many other embodiments. Without departing from the spirit and essence of the present invention, those skilled in the art can make various corresponding changes and modifications according to the present invention, but these corresponding changes and modifications should fall within the protection scope of the claims attached to the present invention.

## Claims

1. A safety syringe, comprising:
an outer barrel comprising a front end opening located in a front end thereof, a first rear end opening located in an opposing rear end thereof, a first annular flange and a second annular flange located on an inner surface of the rear end of said outer barrel, and a receiving groove located on the inner surface of the rear end of said outer barrel between said first annular flange and said second annular flange;
an inner barrel comprising a needle mount located on a front end thereof, a positioning portion adjacent to said needle mount, a second rear end opening located in an opposing rear end thereof; and
a plunger inserted through said second rear end opening of said inner barrel into the inside of said inner barrel and axially movable relative to said inner barrel;
wherein said inner barrel is inserted through said first rear end opening of said outer barrel into the inside of said outer barrel and axially movable relative to said outer barrel between a first position where said positioning portion of said inner barrel is located between said front end opening of said outer barrel and said second annular flange of said outer barrel so that said needle mount of said inner barrel is partially exposed to the outside of said outer barrel through said front end opening and a second position where said positioning portion of said inner barrel is located in said receiving groove of said outer barrel and blocked by said first annular flange and said second annular flange of said outer barrel so that said needle mount of said inner barrel is completely inside said outer barrel.

2. The safety syringe as claimed in claim 1, wherein said first rear end opening of said outer barrel is adjacent to said first annular flange of said outer barrel, and the inner diameter of said first rear end opening of said outer barrel gradually increases in direction away from said first annular flange of said outer barrel.

3. The safety syringe as claimed in claim 1, wherein the inner diameter of said second annular flange of said outer barrel gradually increases in direction away from said receiving groove of said outer barrel.

4. The safety syringe as claimed in claim 1, wherein said positioning portion of said inner barrel is composed of four bumps equiangularly spaced around the central axis of said inner barrel.

5. The safety syringe as claimed in claim 1, wherein said positioning portion of said inner barrel is an annular bump.

6. The safety syringe as claimed in claim 1, wherein the axial length of said receiving groove of said outer barrel is larger than the axial length of said positioning portion of said inner barrel, so that when said inner barrel is at said second position, said outer barrel is bendable relative to said inner barrel, making said front end opening of said outer barrel and said needle mount of said inner barrel staggered from each other without being on the same line.
